# EUROPÄISCHE PATENTSCHRIFT

(11) **EP 2 072 624 B1**
(45) Veröffentlichungstag und Bekanntmachung des Hinweises auf die Patenterteilung: **15.05.2013**
(21) Anmeldenummer: 08020688.1
(22) Anmeldetag: 28.11.2008
(51) Int. Cl.: C12Q 1/56, G01N 33/86

(54) **Langzeitstabiles Thromboplastin - Reagenz**
Long-term stable thromboplastin reagent
Réactif à base de thromboplastine stable à long terme

(30) Priorität: 21.12.2007 DE 102007062323
(43) Veröffentlichungstag der Anmeldung: 24.06.2009
(73) Patentinhaber: Siemens Healthcare Diagnostics Products GmbH, 35041 Marburg (DE)
(72) Erfinder: Rechner, Andreas, 35041 Marburg (DE)

(56) Entgegenhaltungen:
- EP-A- 0 020 895
- US-A- 3 183 159
- US-A- 3 980 432
- US-A- 4 416 812
- US-A1- 2003 157 582
- VOLPI I: "The hemostatic and vasoprotective effect of combined thromboplastin and quercetin in obstetrics and gynecology" MINERVA GINECOLOGICA, TORINO, IT, Bd. 11, Nr. 5, 15. März 1959 (1959-03-15), Seiten 210-212, XP009112448 ISSN: 0026-4784

## Beschreibung

Die vorliegende Erfindung liegt auf dem Gebiet der Gerinnungsdiagnostik und betrifft ein Reagenz auf Basis von rekombinantem oder nativem Gewebefaktor und Phospholipiden, welches durch die Zugabe eines Polyphenols stabilisiert werden kann.

Der Gewebefaktor (Thromboplastin, engl. tissue factor) ist ein Transmembranprotein mit essentieller Bedeutung für die Blutgerinnung. Es wird von Zellen exprimiert, die normalerweise nicht mit fließendem Blut in Kontakt stehen, wie z. B. von Zellen im Subendothel (Glatte Muskulatur) und von Zellen, die Blutgefäße umgeben (z. B. Fibroblasten). Im Falle einer Blutgefäßschädigung geraten die Gewebefaktor-exprimierenden Zellen jedoch in Kontakt mit Faktor VII, einem prokoagulatorischen Blutgerinnungsfaktor, der im Blut zirkuliert. In Gegenwart von Calcium bilden Gewebefaktor und Faktor VII einen Komplex, und die Aktivität von Faktor VII wird vertausendfacht (F VII > F Vlla). Der Komplex aus Gewebefaktor und Faktor VIIa katalysiert in Gegenwart von Phospholipiden und Calcium die Umwandlung des inaktiven Blutgerinnungsfaktors Faktor X in aktivierten Faktor Xa und beschleunigt so den Gerinnungsprozess. Zusammen mit Faktor VII bildet der Gewebefaktor den sogenannten extrinsischen Weg der Blutgerinnung, durch den eine Verletzung der Blutgefäße durch schnellstmögliche Blutgerinnung unterbunden werden soll.

In der Gerinnungsdiagnostik wurden verschiedene in vitro-Testverfahren entwickelt, mit deren Hilfe bestimmt werden kann, ob das Blut oder Plasma eines Patienten einwandfrei gerinnen kann oder ob eine Gerinnungsstörung vorliegt. Im Fall einer Gerinnungsstörung ist es häufig erforderlich, präzisere Kenntnisse über die Ursache der vorliegenden Störung zu erlangen, um die optimalen therapeutischen Maßnahmen auswählen zu können. Zur Untersuchung verschiedener Teilfunktionen der Blutgerinnung, insbesondere zur Untersuchung des extrinsischen Systems der Blutgerinnung, wird Gewebefaktor als Aktivator verwendet. Die bekannteste Anwendung von Gewebefaktor als Gerinnungsaktivator ist der sogenannte Quick-Test zur Bestimmung der Prothrombinzeit (PT) (synonym: Thromboplastinzeit). Im Quick-Test und seinen Varianten wird üblicherweise eine Plasmaprobe mit einer Mischung von Gewebefaktor, Phospholipiden und Calciumionen-vermischt, und es wird die Zeit vom Zeitpunkt des Vermischens bis zur fassbaren Fibrinbildung in Sekunden gemessen. In Gerinnungstesten, bei denen chromogene Substrate verwendet werden, wird alternativ die Zeit vom Zeitpunkt des Vermischens bis zum Erreichen einer bestimmten Absorptionsänderung gemessen. Gewebefaktor wird auch in anderen Testverfahren eingesetzt, die nicht der Bestimmung einer Gerinnungszeit dienen, sondern der Bestimmung einzelner Komponenten des Gerinnungssystems, wie z. B. des endogenen Thrombinpotenzials (ETP) (EP 420 332 A2). Prinzipiell kann Gewebefaktor bei allen Testen verwendet werden, die sich mit Komponenten der extrinsischen Gerinnung beschäftigen.

Das Thromboplastin-Reagenz (Gewebefaktor-Reagenz) hat eine zentrale Bedeutung für den jeweiligen Test. Üblicherweise enthält ein Thromboplastin-Reagenz den Gewebefaktor zusammen mit gerinnungsaktiven Phospholipiden. Der Gewebefaktor wird entweder als Gewebeextrakt aus unterschiedlichen Organen (z. B. Hirn, Plazenta, Lunge) verschiedener Spezies (z. B. Kaninchen, Mensch, Rind) gewonnen oder rekombinant hergestellt. Im Stand der Technik sind zahlreiche Methoden zur Gewinnung von Gewebefaktor und zur Herstellung von Thromboplastin-Reagenzien bekannt, und eine Vielzahl von Thromboplastin-Reagenzien ist kommerziell erhältlich.

US 4 416 812 beschreibt ein entsprechendes Reagenz.

Zurzeit werden die meisten käuflichen Thromboplastin-Reagenzien in gefriergetrockneter Form gehandelt und müssen daher vor Gebrauch in einem Rekonstitutionsmedium, z. B. in destilliertem Wasser oder einer Pufferlösung aufgelöst werden. Der Grund hierfür ist die mangelnde Stabilität der Reagenzien im flüssigen Zustand. Der Nachteil von Reagenzien, die in gefriergetrockneter Form bereit gestellt werden, besteht nicht nur darin, dass Hersteller und Anwender zusätzliche zeit- und kostenintensive Verfahrensschritte durchführen müssen (Lyophilisation und Rekonstitution), sondern auch darin dass diese zusätzlichen Maßnahmen das Risiko bergen, dass es dabei zu Fehlern kommt, die die Qualität des Reagenzes beeinträchtigen können. Wünschenswert sind daher flüssige, gebrauchsfertige Reagenzformulierungen. Ein Problem bei der Bereitstellung flüssiger Thromboplastin-Reagenzien ist jedoch ihre mangelnde Stabilität. Unter der Stabilität eines Thromboplastin-Reagenzes kann z. B. die zeitliche Konstanz der Prothrombinzeit für ein definiertes Plasma, z.-B. ein Normalplasma verstanden werden. Idealerweise sollte ein Thromboplastin-Reagenz über die Dauer seiner Lagerung oder seines Gebrauchs seine Spezifikationen beibehalten, beziehungsweise im günstigsten Fall die Eigenschaften und Charakteristika wie zum Zeitpunkt seiner Produktion.

Im Stand der Technik sind verschiedene Strategien zur Stabilisierung flüssiger Thromboplastin-Reagenzien beschrieben. In EP 942 284 A2 wird ein flüssiges, auf rekombinantem Gewebefaktor basierendes Thromboplastin-Reagenz beschrieben, das durch die kombinierte Zugabe von Ascorbinsäure und einem Serumalbumin stabilisiert wird. In US 3,522,148 wird ein flüssiges, auf aus Gewebe extrahiertem (natürlichem) Gewebefaktor basierendes Thromboplastin-Reagenz beschrieben, das durch Zugabe bestimmter Natrium- oder Calciumsalze stabilisiert wird. In EP 585 987 A1 wird ein anderes flüssiges, auf natürlichem Gewebefaktor basierendes Thromboplastin-Reagenz beschrieben, das durch die Zugabe verschiedener Stabilisatoren, wie Albumin oder Polyethylenglykol, sowie verschiedener antimikrobiell wirksamer Substanzen, wie Natriumazid oder Antibiotika, stabilisiert wird.

Der vorliegenden Erfindung lag die Aufgabe zugrunde, ein alternatives Verfahren zur Stabilisierung eines flüssigen Thromboplastin-Reagenzes bereit zu stellen. Die Lösung dieser Aufgabe besteht in der Bereitstellung der in den Ansprüchen beschriebenen erfindungsgemäßen Verfahren und Gegenständen. Gelöst wird die Aufgabe insbesondere dadurch, dass einem Thromboplastin-Reagenz, das Gewebefaktor und Phospholipide enthält, mindestens ein wasserlösliches Polyphenol zugesetzt wird, welches mindestens eine Catecholfunktion aufweist.

Polyphenole sind aromatische Verbindungen, die zwei oder mehr phenolische Hydroxylgruppen im Molekül enthalten (siehe auch Macheix, Jean-Jaques et al.: Fruit Phenolics. CRC Press, Inc., Boca Raton, USA, 1990, Kapitel 1). Natürliche Polyphenole kommen in Pflanzen als Farbstoffe (Anthocyane), Geschmacksstoffe und Gerbsäuren (Tannine) vor. Weit mehr als 8000 verschiedene dieser meist bioaktiven, so genannten sekundären Pflanzenstoffe sind zurzeit bekannt. Ihre Funktionen in der Pflanze reichen vom Schutz vor Fraßfeinden und dem Befall durch Bakterien oder Pilze bis zur Anlockung von Insekten zur Bestäubung durch ihre Farbe (z. B. in den Blüten).

Unter dem Begriff "Catecholfunktion" ist das Vorhandensein von zwei orthoständigen Hydroxylgruppen an einem aromatischen Ring des Polyphenols zu verstehen, d. h. das Vorhandensein von jeweils einer Hydroxylgruppe an zwei direkt benachbarten C-Atomen eines aromatischen Rings (z. B. in 1,2-Stellung, 2,3-Stellung, 3,4-Stellung u. s. w., wie z. B. in der 3,4-Dihydroxybenzoesäure).

Geeignete Polyphenole im Sinne der vorliegenden Erfindung müssen mindestens eine Catecholfunktion aufweisen. Sie können mehr als eine Catecholfunktion aufweisen, wobei die Catecholfunktionen sowohl an demselben aromatischen Ring als auch an verschiedenen aromatischen Ringen des Polyphenols vorhanden sein können. Beispielsweise können zwei Catecholfunktionen dadurch vorhanden sein, dass ein aromatischer Ring drei ortho-ständige Hydroxyl-Gruppen aufweist (z. B. in 3,4,5-Stellung, wie z: B. Gallocatechin) oder dadurch dass zwei aromatische Ringe der Verbindung jeweils eine Catecholfunktion aufweisen (wie z. B. Rosmarinsäure). Drei Catecholfunktionen können beispielweise dadurch vorhanden sein, dass ein erster aromatischer Ring drei ortho-ständige Hydroxyl-Gruppen aufweist und ein zweiter aromatischer Ring zwei ortho-ständige Hydroxyl-Gruppen aufweist (wie z. B. Catechingallat) oder dadurch dass drei aromatische Ringe der Verbindung jeweils eine Catecholfunktion aufweisen (wie z. B. Procyanidin-Trimer aus drei Catechinmolekülen). Entsprechendes gilt für Polyphenole, die mehr als drei Catecholfunktionen aufweisen. Epigallocatechingallat verfügt beispielweise über vier Catecholfunktionen, und zwar dadurch dass zwei aromatische Ringe jeweils drei ortho-ständige Hydroxyl-Gruppen aufweisen.

Die Flavonoide und die Phenolsäuren bilden die zwei wichtigsten Subgruppen der Polyphenole. Zusätzlich werden noch die Stoffgruppen der Stilbene, Coumarine, Xanthone, Lignine und Tannine zu den Polyphenolen gezählt. Die Flavonoide sind eine Gruppe von wasserlöslichen Pflanzenfarbstoffen, die chemisch alle auf dem Flavan-Grundgerüst aufbauen (siehe auch Macheix, Jean-Jaques et al.: Fruit Phenolics. CRC Press, Inc., Boca Raton, USA, 1990, Kapitel 1). Die meisten Flavonoide sind an Glukose oder Rhamnose gebunden, weswegen man sie auch Glykoside nennt. Nur die Flavan-3-ole und die Proanthocyanidine sind nicht an Zuckermoleküle gebunden (= Aglykone).

Die Flavonoide werden nach strukturellen Eigenschaften weiter eingeteilt in:
- Flavonole (z. B. Quercetin, Rutin, Kaempferol, Myricetin und deren Derivate),
- Flavan-3-ole (z. B. Catechin, Gallocatechin, Epicatechin, Epigallocatechingallat),
- Procyanidin (polymere Flavan-3-ole),
- Flavone (z. B. Luteolin, Apigenin, Morin und deren Derivate),
- Flavanone (z. B. Hesperetin, Naringenin, Eriodictyol und deren Derivate, wie z. B. Hesperidin oder Naringin),
- Isoflavone (z. B. Genistein, Daidzein und deren Derivate),
- Anthocyane (z. B. Cyanidin, Delphinidin, Malvidin, Pelargonidin, Peonidin, Petunidin und deren Derivate).

Es wurde beschrieben, dass galloylierte Flavan-3-ole wie Epigallocatechingallat in vitro inhibierend auf die Thrombingenerierung wirken [Stampfuss et al. (2005) Green tea catechins containing a galloyl group in the 3' position inhibit tissue factor induced thrombin generation. Thromb Haemost 93: 1200-1201].

Die Phenolsäuren sind chemische Verbindungen, die sich in die Gruppen der Hydroxybenzoesäuren und der Hydroxyzimtsäuren aufteilen (siehe auch Macheix, Jean-Jaques et al.: Fruit Phenolics. CRC Press, Inc., Boca Raton, USA, 1990, Kapitel 1). Zu den Hydroxybenzoesäuren zählen u. a. die Salicylsäure, die Gallussäure, die Protocatechuesäure und die Vanillinsäure sowie deren Derivate, wie z. B. die 3,4-Dihydroxybenzoesäure. Zu den bekanntesten Hydroxyzimtsäuren gehören z. B. die Kaffeesäure, die Ferulasäure, die Sinapinsäure und die Coumarsäure sowie deren Derivate, wie z. B. die Caffeoylglucose, die Rosmarinsäure, die Ferulylchinasäure. In der Natur kommen die Phenolsäuren ähnlich wie die Flavonoide fast ausschließlich in gebundener Form vor. Am häufigsten sind Ester mit organischen Säuren oder Glykosiden, wie z. B. die Chlorogensäure oder die Caftarsäure.

Gegenstand der vorliegenden Erfindung ist ein Verfahren zur Stabilisierung von Thromboplastin-Reagenzien, d. h. von wässrigen Lösungen, die Gewebefaktor sowie Phospholipide enthalten. Es wurde gefunden, dass die Zugabe eines oder mehrerer Polyphenole, wobei mindestens ein Polyphenol wasserlöslich ist und mindestens eine Catecholfunktion aufweist, die Stabilität einer wässrigen Lösung, die Gewebefaktor und Phospholipide enthält, erhöht. Erfindungsgemäß stabilisierte Thromboplastin-Reagenzien liefern noch nach 12-monatiger Lagerung bei +2 °C bis +8 °C Prothrombinzeit- (PT-) Messergebnisse, die weniger als 25 % von dem PT-Messergebnis zu Beginn der Laufzeit abweichen. Eine forcierte Lagerung bei +37 °C über 8 Wochen dient der schnellen Abschätzung, ob eine Lagerungsstabilität bei +2 °C bis +8 °C über einen Zeitraum von 12 oder mehr Monaten erwartet werden kann. Liefert ein stabilisiertes Thromboplastin-Reagenz bei einer Lagerung bei +37 °C über einen Zeitraum von 4 Wochen Prothrombinzeit- (PT-) Messergebnisse, die weniger als 25 % von dem PT-Messergebnis zu Beginn der Laufzeit abweichen, kann erwartet werden, dass das Reagenz bei einer Lagerungstemperatur von +2 °C bis +8 °C über einen Zeitraum von 12 oder mehr Monaten stabil ist.

Im erfindungsgemäßen Verfahren wird dem Thromboplastin-Reagenz mindestens ein wasserlösliches Polyphenol, das mindestens eine Catecholfunktion aufweist, zugesetzt, so dass das Polyphenol in einer Endkonzentration von 0,15 bis 10 mM, besonders bevorzugt von 0,5 bis 2 mM im Reagenz enthalten ist. (Siehe Ansprüche).

In einer speziellen Ausführungsform des erfindungsgemäßen Verfahrens werden dem Thromboplastin-Reagenz zwei oder mehr wasserlösliche Polyphenole zugesetzt, von denen mindestens eines mindestens eine Catecholfunktion aufweist. Vorzugsweise werden dem Thromboplastin-Reagenz zwei wasserlösliche Polyphenole zugesetzt, wobei beide Polyphenole mindestens eine Catecholfunktion aufweisen. Bevorzugterweise werden dem Thromboplastin-Reagenz die beiden oder mehr Polyphenole zugesetzt, so dass jedes der Polyphenole-in einer Endkonzentration von 0,15 bis 10 mM, besonders bevorzugt von 0,5 bis 2 mM im Reagenz enthalten ist. Bevorzugte-Kombinationen mehrerer Polyphenole umfassen die Kombination von Chlorogensäure und Catechin oder die Kombination von Chlorogensäure und Rutin.

In einer speziellen Ausführungsform des erfindungsgemäßen Verfahrens wird mindestens ein wasserlösliches Polyphenol zur Stabilisierung verwendet, welches mindestens eine Catecholfunktion aufweist und aus der Gruppe der Flavonoide stammt. Geeignete Flavonoide, die wasserlöslich sind und mindestens eine Catecholfunktion aufweisen, sind beispielsweise:
- Quercetin, Rutin, Myricetin und deren Glykoside, die zur Subgruppe der Flavonole gehören;
- Catechin, Gallocatechin, Epicatechin, Epigallocatechingallat, die zur Subgruppe der Flavan-3-ole gehören;
- Procyanidine, die zur Subgruppe der polymeren Flavan-3-ole gehören;
- Luteolin und dessen Glykoside, die zur Subgruppe der Flavone gehören; und
- Cyanidin, Delphinidin, Petunidin und deren Glykoside, die zur Subgruppe der Anthocyane gehören.

In einer besonders bevorzugten Ausführungsform des erfindungsgemäßen Verfahrens wird mindestens Catechin oder Epicatechin zur Stabilisierung verwendet.

In einer weiteren speziellen Ausführungsform des erfindungsgemäßen Verfahrens wird mindestens ein wasserlösliches Polyphenol zur Stabilisierung verwendet, welches mindestens eine Catecholfunktion aufweist und aus der Gruppe der Phenolsäuren stammt. Geeignete Phenolsäuren, die wasserlöslich sind und mindestens eine Catecholfunktion aufweisen, sind beispielsweise:
- Gallussäure, Protocatechuesäure (3,4-Dihydroxybenzoesäure) sowie deren Ester oder Glykoside, die zur Subgruppe der Hydroxybenzoesäuren gehören; und
- Kaffeesäure, Chlorogensäure, Caftarsäure, Caffeoylglucose, Rosmarinsäure sowie deren Ester oder Glykoside, die zur Subgruppe der Hydroxyzimtsäuren gehören.
   In einer besonders bevorzugten Ausführungsform des erfindungsgemäßen Verfahrens wird mindestens Chlorogensäure zur Stabilisierung verwendet.

In einer weiteren Ausführungsform des erfindungsgemäßen Verfahrens wird dem Thromboplastin-Reagenz zur weiteren Stabilisierung zusätzlich L-Ascorbinsäure und/oder Polyethylenglykol und/oder eine antimikrobiell wirksame Substanz zugesetzt. Bevorzugterweise wird so viel L-Ascorbinsäure zugesetzt, dass sie in einer Endkonzentration von 0,1 bis 10 mM, besonders bevorzugt von 0,5 bis 1 mM im Reagenz enthalten ist. Bevorzugterweise wird so viel Polyethylenglykol zugesetzt, dass es in einer Endkonzentration von 0,1 bis 5 % (w/v), besonders bevorzugt von 0,25 bis 1 % (w/v) im Reagenz enthalten ist. Besonders bevorzugte antimikrobiell wirksame Substanzen sind Natriumazid und Thymol. Bevorzugterweise wird so viel Natriumazid zugesetzt, dass es in einer Endkonzentration von 0,01 bis 1 % (w/v), besonders bevorzugt von 0,1 % (w/v) im Reagenz enthalten ist.

Das erfindungsgemäße Verfahren eignet sich sowohl zur Stabilisierung von Thromboplastin-Reagenzien, die rekombinanten Gewebefaktor (human oder tierisch, insbesondere Kaninchen) zusammen mit natürlichen und/oder synthetischen Phospholipiden enthalten, als auch zur Stabilisierung von Thromboplastin-Reagenzien, die natürlichen humanen oder tierischen (z. B. Kaninchen, Rind) Gewebefaktor aus Gewebeextrakten (z. B. aus Hirn, Plazenta, Lunge) zusammen mit natürlichen und/oder synthetischen Phospholipiden enthalten.

Ein weiterer Gegenstand der vorliegenden Erfindung ist ein Reagenz bzw. eine wässrige Lösung, die sich zur Verwendung als Thromboplastin-Reagenz eignet, die Gewebefaktor und Phospholipide enthält sowie mindestens ein wasserlösliches Polyphenol, welches mindestens eine Catecholfunktion aufweist. Bevorzugterweise ist das mindestens eine Polyphenol in einer Endkonzentration von 0,15 bis 10 mM, besonders bevorzugt 0,5 bis 2 mM in dem Reagenz enthalten. Eine bevorzugte Ausführungsform eines erfindungsgemäßen Reagenzes enthält zusätzlich Calciumionen. Weitere erfindungsgemäße Ausführungsformen des Reagenzes sind durch die oben beschriebenen diversen Ausführungsformen des erfindungsgemäßen Verfahrens erhältlich. Das Reagenz kann als Flüssigreagenz oder auch als in Wasser oder Puffer rekonstituierbares Lyophilisat bereitgestellt werden.

Ein weiterer Gegenstand der vorliegenden Erfindung ist die Verwendung eines erfindungsgemäßen Thromboplastin-Reagenzes in einem in vitro-Verfahren zur Bestimmung eines Gerinnungsparameters in einer Patientenprobe, inbesondere zur Bestimmung eines Gerinnungsparameters aus der Gruppe Prothrombinzeit (PT, auch Quick-Test) und deren Varianten und Endogenes Thrombinpotenzial (ETP). Das erfindungsgemäße Reagenz eignet sich zur Verwendung als Aktivator der Gerinnungskaskade, beispielsweise in Testverfahren, die auf der Detektion eines Fibringerinnsels beruhen wie auch in chromogenen oder fluorogenen Testverfahren.

Ein weiterer Gegenstand der vorliegenden Erfindung ist ein Testkit, insbesondere zur Durchführung eines vitro-Verfahrens zur Bestimmung eines Gerinnungsparameters, wobei das Testkit mindestens ein erfindungsgemäß stabilisiertes Thromboplastin-Reagenz enthält. Für den Fall, dass das erfindungsgemäße Thromboplastin-Reagenz als Lyophilisat in dem Kit enthalten ist, enthält das Kit bevorzugterweise ein geeignetes Rekonstitutionsmedium (z. B. destilliertes Wasser oder eine Pufferlösung). Bevorzugt ist ein Testkit, das ein Behältnis mit einem flüssigen Thromboplastin-Reagenz enthält.

### Figurenbeschreibung

**Figur 1**
   Prothrombinzeiten eines definierten Referenzplasmas (Kalibrator 4) mit verschiedenartig stabilisierten Thromboplastin-Reagenzien während einer Lagerung von 12 Monaten bei +2 bis +8 °C (siehe Beispiel 1), (PEG = Polyethylenglykol;-Azid = Natriumazid).
**Figur 2**
   Prothrombinzeiten eines definierten Referenzplasmas (Kalibrator 1) mit verschiedenartig stabilisierten Thromboplastin-Reagenzien während einer Lagerung von 42 Wochen bei +2 bis +8 °C (siehe Beispiel 2), (Cat = Catechin; Asc = Ascorbinsäure; Azid = Natriumazid).
**Figur 3**
   Prothrombinzeiten eines definierten Referenzplasmas (Kalibrator 1) mit verschiedenartig stabilisierten Thromboplastin-Reagenzien während einer Lagerung von 8 Wochen bei +37 °C (siehe Beispiel 3), (Azid = Natriumazid).
**Figur 4**
   Prothrombinzeiten eines definierten Referenzplasmas (Kalibrator 1) mit verschiedenartig stabilisierten Thromboplastin-Reagenzien während einer Lagerung von 8 Wochen bei +37 °C (siehe Beispiel 4), (Azid = Natriumazid).
**Figur 5**
   Prothrombinzeiten definierter Referenzplasmen (K1 bis K6, PT Multi Calibrator Kit, Dade Behring Marburg GmbH) mit verschiedenartig stabilisierten Thromboplastin-Reagenzien während einer Lagerung von 8 Wochen bei +37 °C (siehe Beispiel 5). Die folgenden Ausführungsbeispiele dienen der Veranschaulichung der vorliegenden Erfindung und sind nicht als Einschränkung zu verstehen.

### Beispiel 1: Bestimmung der Stabilität eines mit Catechin stabilisierten Thromboplastin-Reagenzes während einer 12-monatigen Lagerung bei +2 - +8 °C

Ein lyophilisiertes Thromboplastin-Reagenz, das rekombinanten humanen Gewebefaktor, synthetische Phospholipide und Calciumionen enthielt, wurde zum Zeitpunkt t₀ mit destilliertem Wasser rekonstituiert, und dem gelösten Reagenz wurden folgende Substanzen in der angegebenen Endkonzentration zugesetzt:

| | | | |
|---|---|---|---|
| 1. kein weiterer Zusatz | | | |
| 2. 0.1 % (w/v) Natriumazid | | | |
| 3. 0.1 % (w/v) Natriumazid + | 1 % (w/v) PEG | | |
| 4. 0.1 % (w/v) Natriumazid + | 1 % (w/v) PEG | + | 2 mM Ascorbinsäure |
| 5. 0.1 % (w/v) Natriumazid + | 1 % (w/v) PEG | + | 1 mM Catechin |
| 6. 0.1 % (w/v) Natriumazid + | | | 1 mM Catechin |

Die verschiedenen Reagenzien wurden in einem automatischen Prothrombinzeit-Test (PT-Test) in einem BCT®-Gerinnungsmessgerät (Behring Coagulation Timer, Dade Behring Marburg GmbH, Marburg, Deutschland) eingesetzt. Als Probe wurde ein definiertes Referenzplasma (Kalibrator 4 des Kits PT-Multi Calibrator, Dade Behring Marburg GmbH, Marburg, Deutschland) verwendet. Probe und Reagenz wurden jeweils auf 37 °C vorgewärmt und schließlich vermischt. Durch die Zugabe des Reagenzes wurde der Gerinnungsvorgang ausgelöst, und es wurde die Zeit bis zur Bildung des Fibringerinnsels gemessen.

Zur Bestimmung der Langzeitstabilität der verschiedenen Reagenzien wurden die Reagenzien in flüssigem Zustand bei +2 bis +8°C über einen Zeitraum von bis zu 12 Monaten gelagert. Monatlich wurden Proben der Reagenzien entnommen, und es wurde die Prothrombinzeit desselben Referenzplasmas bestimmt. Als Kontrolle wurde zu jedem Zeitpunkt lyophilisiertes Thromboplastin-Reagenz derselben Charge, die für die Herstellung der verschiedenen Reagenzien verwendet worden war, mit destilliertem Wasser frisch rekonstituiert und gemessen.

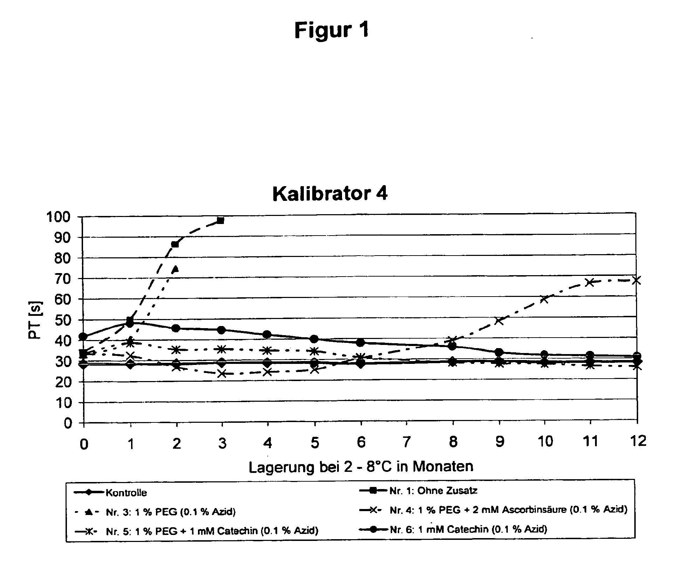

In Figur 1 sind die Prothrombinzeiten der verschiedenen Thromboplastin-Reagenzien über einen Zeitraum von 12 Monaten gezeigt. Stabile Prothrombinzeiten mit Abweichungen von weniger als 25 % gegenüber dem Ausgangswert zum Zeitpunkt t₀ wurden nur mit den erfindungsgemäßen, mit Catechin stabilisierten Thromboplastin-Reagenzien (Nr. 5 und 6) erreicht. Alle anderen Reagenzien lieferten spätestens nach 9 Monaten (siehe Nr. 4) nur noch stark abweichende Prothrombinzeiten. Das Natriumazid hatte auf die Leistungsstabilität des Reagenz keinerlei Einfluss. Reagenz Nr. 2 (0,1 % Natriumazid; Werte nicht gezeigt) verhielt sich wie Reagenz Nr. 1 (kein Zusatz). Allerdings zeigte sich Reagenz Nr. 2 (0,1 % Natriumazid) deutlich weniger trüb als Reagenz Nr. 1 (ohne Natriumazid).

### Beispiel 2: Bestimmung der Stabilität mit Catechin stabilisierter Thromboplastin-Reagenzien während einer 48-wöchigen Lagerung bei 2 - 8 °C

Dem gleichen Thromboplastin-Reagenz wie in Beispiel 1 beschrieben wurden folgende Substanzen in der angegebenen Endkonzentration zugesetzt:
7. 0.1 % (w/v) Natriumazid + 1 mM Catechin
8. 0.1 % (w/v) Natriumazid + 0,5 mM Catechin
9. 0.1 % (w/v) Natriumazid + 0,1 mM Catechin
10. 0.1 % (w/v) Natriumazid + 0,5 mM Catechin + 1 mM L-Ascorbinsäure

Die verschiedenen Reagenzien wurden, wie in Beispiel 1 beschrieben, zur PT-Bestimmung eines definierten Referenzplasmas (Kalibrator 1 des Kits PT-Multi-Calibrator, Dade Behring Marburg GmbH, Marburg, Deutschland) eingesetzt.

Zur Bestimmung der Langzeitstabilität der verschiedenen Reagenzien wurden die Reagenzien in flüssigem Zustand bei +2 bis +8 °C über einen Zeitraum von bis zu 48 Wochen gelagert. Vierwöchentlich wurden Proben der Reagenzien entnommen, und es wurde die Prothrombinzeit derselben Referenzplasmen bestimmt. Als Kontrolle wurde zu jedem Zeitpunkt lyophilisiertes Thromboplastin-Reagenz derselben Charge, die für die Herstellung der verschiedenen Reagenzien verwendet worden war, mit destilliertem Wasser frisch rekonstituiert und gemessen.

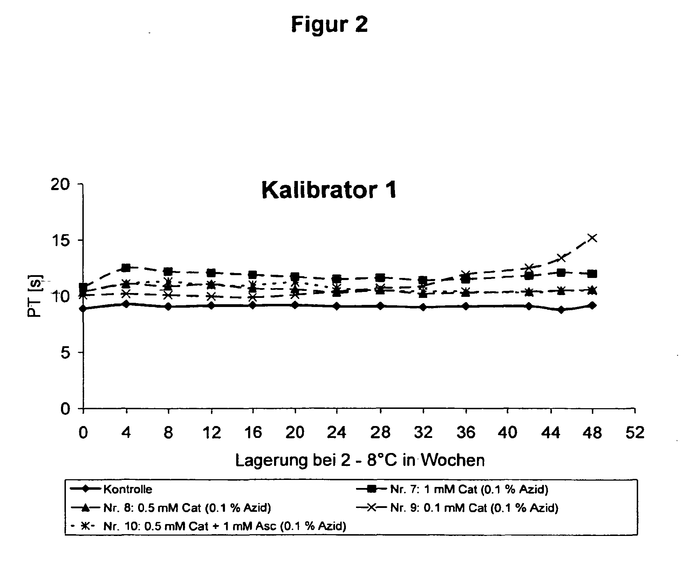

Der Lagerversuch zeigt, dass eine Konzentration von 0,1 mM Catechin (Reagenz Nr. 9) nicht ausreichend ist, um das Thromboplastin-Reagenz für mehr als ca. 32 Wochen bei +2 - +8 °C zu stabilisieren (Figur 2). Eine Konzentration von 0,5 mM Catechin sowie die Kombination aus 0,5 mM Catechin und 1 mM L-Ascorbinsäure bewirken jedoch eine Stabilisierung des Thromboplastin-Reagenzes über einen Zeitraum von mindestens 48 Wochen (Figur 2). Das Ausmaß der beobachteten Verlängerung der Prothrombinzeit nach Zugabe von Catechin ist offensichtlich konzentrationsabhängig.

### Beispiel 3: Bestimmung der Stabilität von mit verschiedenen Polyphenolen stabilisierten Thromboplastin-Reagenzien während einer 8-wöchigen Lagerung bei 37 °C

Dem gleichen Thromboplastin-Reagenz, wie in Beispiel 1 beschrieben, wurden folgende Substanzen in der angegebenen Endkonzentration zugesetzt:
11. 0.1 % (w/v) Natriumazid + 1 mM Catechin
12. 0.1 % (w/v) Natriumazid + 1 mM Epicatechin
13. 0.1 % (w/v) Natriumazid + 1 mM Rutin
14. 0.1 % (w/v) Natriumazid + 1 mM Chlorogensäure
15. 0.1 % (w/v) Natriumazid + 2 mM Chlorogensäure

Die verschiedenen Reagenzien wurden, wie in Beispiel 1 beschrieben, zur PT-Bestimmung eines definierten Referenzplasmas (Kalibrator 1 des Kits PT-Multi Calibrator, Dade Behring) eingesetzt.

Zur Bestimmung der Langzeitstabilität der verschiedenen Reagenzien wurden die Reagenzien in flüssigem Zustand bei +37 °C über einen Zeitraum von bis zu acht Wochen gelagert. Nach einer, vier und acht Wochen wurden Proben der Reagenzien entnommen, und es wurden die Prothrombinzeiten desselben Referenzplasmas bestimmt. Als Kontrolle wurde zu jedem Zeitpunkt lyophilisiertes Thromboplastin-Reagenz derselben Charge, die für die Herstellung der verschiedene Reagenzien verwendet worden war, mit destilliertem Wasser frisch rekonstituiert und gemessen.

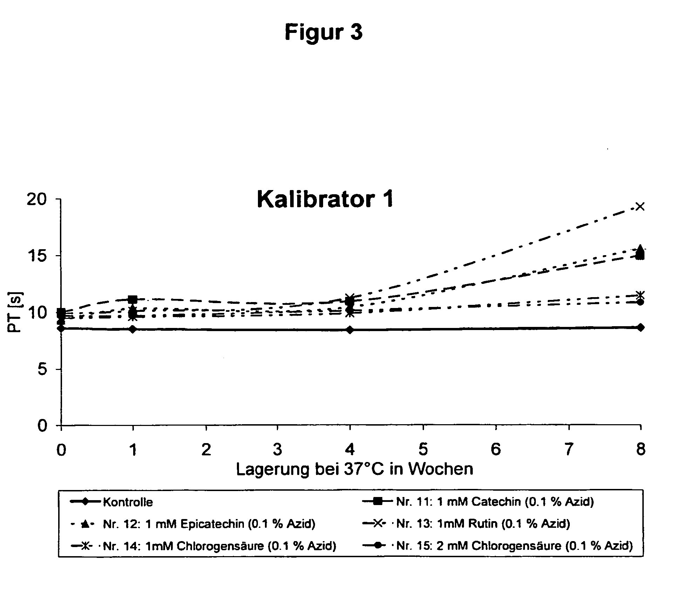

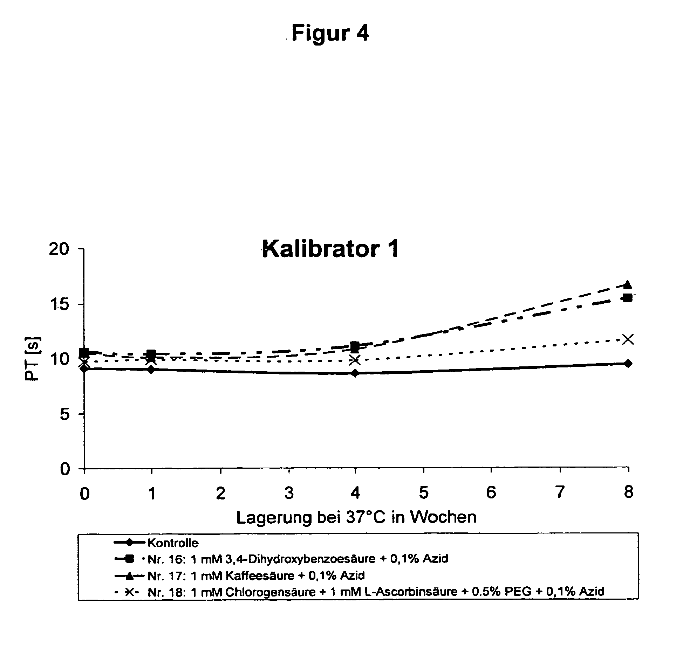

Die forcierte Stabilitätsuntersuchung bei +37 °C zeigt, dass sich das Flavan-3-ol Epicatechin (Epimer des Catechin), das Flavonolglykosid Rutin, und das Hydroxyzimtsäurederivat Chlorogensäure ebenfalls in vergleichbaren Konzentrationen wie Catechin zur Stabilisierung eines Thromboplastin-Reagenzes eignen (Figur 3). Besonders gut konnte das Thromboplastin-Reagenz durch Zugabe von 2 mM Chlorogensäure stabilisiert werden.

### Beispiel 4: Bestimmung der Stabilität von mit verschiedenen Polyphenolen stabilisierten Thromboplastin-Reagenzien während einer 8-wöchigen Lagerung bei 37 °C

Dem gleichen Thromboplastin-Reagenz, wie in Beispiel 1 beschrieben, wurden folgende Substanzen in der angegebenen Endkonzentration zugesetzt:
16. 0.1 % (w/v) Natriumazid + 1 mM 3,4-Dihydroxybenzoesäure
17. 0.1 % (w/v) Natriumazid + 1 mM Kaffeesäure
18. 0.1 % (w/v) Natriumazid + 1 mM Chlorogensäure + 1 mM L-Ascorbinsäure + 0,5 % PEG

Die verschiedenen Reagenzien wurden, wie in Beispiel 1 beschrieben, zur PT-Bestimmung eines definierten Referenzplasmas (Kalibrator 1 des Kits PT-Multi Calibrator, Dade Behring) eingesetzt.

Zur Bestimmung der Langzeitstabilität der verschiedenen Reagenzien wurden die Reagenzien in flüssigem Zustand bei +37 °C über einen Zeitraum von bis zu acht Wochen gelagert. Nach einer, vier und acht Wochen wurden Proben der Reagenzien entnommen, und es wurden die Prothrombinzeiten desselben Referenzplasmas bestimmt. Als Kontrolle wurde zu jedem Zeitpunkt lyophilisiertes Thromboplastin-Reagenz derselben Charge, die für die Herstellung der verschiedenen Reagenzien verwendet worden war, mit destilliertem Wasser frisch rekonstituiert und gemessen.

Die forcierte Stabilitätsuntersuchung bei +37 °C zeigt, dass sich die Polyphenole 3,4-Dihydroxybenzoesäure, Kaffeesäure und Chlorogensäure (Nr. 16 - 18) in den verwendeten Konzentrationen zur Langzeitstabilisierung des lyophilisierten Thromboplastin-Reagenzes eignen. Besonders gut konnte das Thromboplastin-Reagenz durch Zugabe der Substanzkombination (Nr. 18) stabilisiert werden.

### Beispiel 5: Bestimmung der Stabilität von mit mehreren Polyphenolen stabilisierten Thromboplastin-Reagenzien während einer 8-wöchigen Lagerung bei 37 °C

Dem gleichen Thromboplastin-Reagenz, wie in Beispiel 1 beschrieben, wurden folgende Substanzen in der angegebenen Endkonzentration zugesetzt:
19. 0.1 % (w/v) Natriumazid + 0.5 mM Catechin + 0.5 mM Chlorogensäure
20. 0.1 % (w/v) Natriumazid + 1 mM Chlorogensäure + 0.5 mM Rutin
21. 0.1 % (w/v) Natriumazid + 0.5 mM Chlorogensäure + 0.5 mM Catechin + 0.5 mM L-Ascorbinsäure

Die verschiedenen Reagenzien wurden, wie in Beispiel 1 beschrieben, zur PT-Bestimmung definierter Referenzplasmen (K1 bis K6, PT-Multikalibratoren, Dade Behring) eingesetzt. Die daraus resultieren Kalibrationskurven wurden miteinander verglichen.

Zur Bestimmung der Langzeitstabilität der verschiedenen Reagenzien wurden die Reagenzien in flüssigem Zustand bei +37 C über einen Zeitraum von bis zu acht Wochen gelagert. Nach vier und acht Wochen wurden Proben der Reagenzien entnommen, und es wurden die Prothrombinzeiten derselben Referenzplasmen bestimmt. Als Kontrolle wurde zu jedem Zeitpunkt lyophilisiertes Thromboplastin-Reagenz derselben Charge, das für die Herstellung der verschiedenen Reagenzien verwendet worden war, mit destilliertem Wasser frisch rekonstituiert und gemessen.

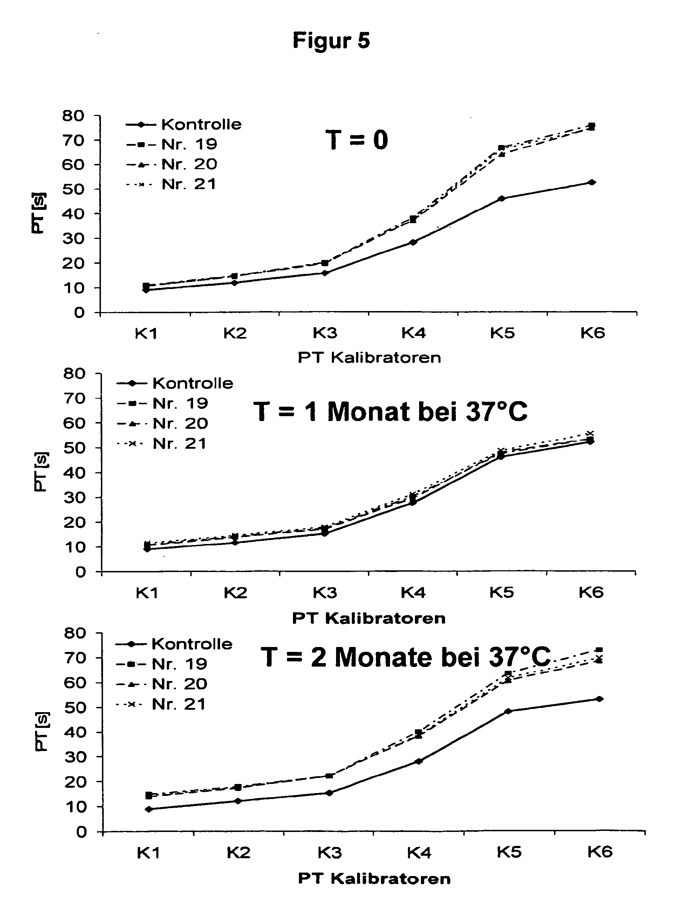

Die forcierte Stabilitätsuntersuchung bei +37 °C zeigt, dass sich Kombinationen der Polyphenole Catechin, Chlorogensäure und Rutin (Nr. 19 - 21) in den verwendeten Konzentrationen zur Langzeitstabilisierung des lyophilisierten Thromboplastin-Reagenzes eignen (Figur 5).

## Patentansprüche

1. Reagenz enthaltend Gewebefaktor und Phospholipide **dadurch gekennzeichnet, dass** es mindestens ein wasserlösliches Polyphenol, welches mindestens eine Catecholfunktion aufweist, in einer Konzentration von 0,15 bis 10 mM, besonders bevorzugt 0,5 bis 2 mM enthält.

2. Reagenz gemäß Anspruch 1 **dadurch gekennzeichnet, dass** es zwei oder mehr wasserlösliche Polyphenole enthält, von denen mindestens eines mindestens eine Catecholfunktion aufweist.

3. Reagenz gemäß einem der vorhergehenden Ansprüche **dadurch gekennzeichnet, dass** es mindestens ein wasserlösliches Polyphenol aus der Gruppe der Flavonoide enthält, welches mindestens eine Catecholfunktion aufweist.

4. Reagenz gemäß Anspruch 3 **dadurch gekennzeichnet, dass** es ein Flavonoid aus der Gruppe Quercetin, Rutin, Myricetin, Catechin, Epicatechin, Gallocatechin, Epigallocatechin, Epigallocatechingallat, Luteolin, Delphinidin, Petunidin und Cyanidin enthält.

5. Reagenz gemäß einem der vorhergehenden Ansprüche **dadurch gekennzeichnet, dass** es mindestens ein wasserlösliches Polyphenol aus der Gruppe der Phenolsäuren enthält, welches mindestens eine Catecholfunktion aufinreist.

6. Reagenz gemäß Anspruch 5 **dadurch gekennzeichnet, dass** es eine Phenolsäure aus der Gruppe Gallussäure, Protocatechuesäure, Kaffeesäure, Rosmarinsäure, Chlorogensäure und Caftarsäure enthält.

7. Reagenz gemäß einem der vorhergehenden Ansprüche **dadurch gekennzeichnet, dass** es zusätzlich L-Ascorbinsäure enthält.

8. Reagenz gemäß einem der vorhergehenden Ansprüche **dadurch gekennzeichnet, dass** es zusätzlich Polyethylenglykol enthält.

9. Reagenz gemäß einem der vorhergehenden Ansprüche **dadurch gekennzeichnet, dass** es zusätzlich mindestens eine antimikrobiell wirksame Substanz enthält.

10. Reagenz gemäß Anspruch 9 **dadurch gekennzeichnet, dass** es eine antimikrobiell wirksame Substanz aus der Gruppe Natriumazid und Thymol enthält.

11. Reagenz gemäß einem der vorhergehenden Ansprüche enthaltend humanen rekombinanten Gewebefaktor, tierischen rekombinanten Gewebefaktor, rekombinanten Kaninchen-Gewebefaktor oder natürlichen humanen oder tierischen Gewebefaktor aus einem Gewebeextrakt.

12. Verwendung eines Reagenzes gemäß einem der vorherigen Ansprüche in einem in vitro-Verfahren zur Bestimmung eines Gerinnungsparameters aus der Gruppe Prothrombinzeit (PT) und Endogenes Thrombinpotenzial (ETP).

13. Testkit zur Durchführung eines vitro-Verfahrens zur Bestimmung eines Gerinnungsparameters **dadurch gekennzeichnet, dass** es ein Reagenz gemäß einem der Ansprüche 1-11 enthält.

14. Verfahren zur Stabilisierung eines Reagenzes enthaltend Gewebefaktor und Phospholipide **dadurch gekennzeichnet, dass** dem Reagenz mindestens ein wasserlösliches Polyphenol, welches mindestens eine Catecholfunktion aufweist, zugesetzt wird, so dass es in einer Endkonzentration von 0,15 bis 10 mM, besonders bevorzugt 0,5 bis 2 mM, im Reagenz enthalten ist.

## Claims

1. A reagent comprising tissue factor and phospholipids which comprises at least one water-soluble polyphenol which has at least one catechol function, in a concentration of from 0.15 to 10 mM, particularly preferably 0.5 to 2 mM.

2. The reagent as claimed in claim 1, which comprises two or more water-soluble polyphenols, of which at least one has at least one catechol function.

3. The reagent as claimed in either of the preceding claims, which comprises at least one water-soluble polyphenol from the group of flavonoids which has at least one catechol function.

4. The reagent as claimed in claim 3, which comprises a flavonoid from the group of quercetin, rutin, myricetin, catechin, epicatechin, gallocatechin, epigallocatechin, epigallocatechin gallate, luteolin, delphinidin, petunidin and cyanidin.

5. The reagent as claimed in any of the preceding claims, which comprises at least one water-soluble polyphenol from the group of phenolic acids which has at least one catechol function.

6. The reagent as claimed in claim 5, which comprises a phenolic acid from the group of gallic acid, protocatechuic acid, caffeic acid, rosmarinic acid, chlorogenic acid and caftaric acid.

7. The reagent as claimed in any of the preceding claims, which additionally comprise L-ascorbic acid.

8. The reagent as claimed in any of the preceding claims, which additionally comprises polyethylene glycol.

9. The reagent as claimed in any of the preceding claims, which additionally comprises at least one substance having an antimicrobial activity.

10. The reagent as claimed in claim 9, which comprises a substance having antimicrobial activity from the group of sodium azide and thymol.

11. The reagent as claimed in any of the preceding claims, comprising human recombinant tissue factor, animal recombinant tissue factor, recombinant rabbit tissue factor or natural human or animal tissue factor from a tissue extract.

12. The use of a reagent as claimed in any of the preceding claims in an in vitro method for determining a coagulation parameter from the group of prothrombin time (PT) and endogenous thrombin potential (ETP).

13. A test kit for carrying out an in vitro method for determining a coagulation parameter, which comprises a reagent as claimed in any of claims 1-11.

14. A method for stabilizing a reagent comprising tissue factor and phospholipids, which comprises adding at least one water-soluble polyphenol which has at least one catechol function to the reagent in such a way that it is present in the reagent in a final concentration of from 0.15 to 10 mM, particularly preferably 0.5 to 2 mM.

## Revendications

1. Réactif contenant du facteur de tissu et des phospholipides, **caractérisé en ce qu'**il contient au moins un polyphénol soluble dans l'eau, qui a au moins une fonction catéchine en une concentration de 0,15 à 10 mM, d'une manière particulièrement préférée de 0,5 à 2 mM.

2. Réactif suivant la revendication 1, **caractérisé en ce qu'**il contient deux ou plusieurs polyphénols solubles dans l'eau, dont au moins l'un a au moins une fonction catéchine.

3. Réactif suivant l'une des revendications précédentes, **caractérisé en ce qu'**il contient au moins un polyphénol soluble dans l'eau du groupe des flavonoïdes, qui a au moins une fonction catéchine.

4. Réactif suivant la revendication 3, **caractérisé en ce qu'**il contient un flavonoïde du groupe de la quercétine, de la rutine, de la myricétine, de la catéchine, de l'épicatéchine, de la gallocatéchine, de l'épigallocatéchine, du gallate d'épigallocatéchine, de la lutéoline, de la delphinidine, de la pétunidine et de la cyanidine.

5. Réactif suivant l'une des revendications précédentes, **caractérisé en ce qu'**il contient au moins un polyphénol soluble dans l'eau du groupe des acides phénoliques, qui a au moins une fonction catéchine.

6. Réactif suivant la revendication 5, **caractérisé en ce qu'**il contient un acide phénolique du groupe de l'acide gallique, de l'acide protocatéchique, de l'acide caféique, de l'acide romarinique, de l'acide chlorogénique et de l'acide caftarique.

7. Réactif suivant l'une des revendications précédentes, **caractérisé en ce qu'**il contient, en outre, de l'acide L-ascorbique.

8. Réactif suivant l'une des revendications précédentes, **caractérisé en ce qu'**il contient, en outre, du polyéthylène glycol.

9. Réactif suivant l'une des revendications précédentes, **caractérisé en ce qu'**il contient, en outre, au moins une substance antimicrobienne.

10. Réactif suivant la revendication 9, **caractérisé en ce qu'**il contient une substance antimicrobienne du groupe du trinitrure de sodium et du thymol.

11. Réactif suivant l'une des revendications précédentes, contenant du facteur de tissu humain recombinant, du facteur de tissu animal recombinant, du facteur de tissu de lapin recombinant ou du facteur de tissu naturel humain ou animal d'un extrait de tissu.

12. Utilisation d'un réactif suivant l'une des revendications précédentes dans un procédé in vitro de détermination d'un paramètre de coagulation du groupe du temps de prothrombine (PT) et du potentiel de thrombine endogène (ETP).

13. Trousse de test pour effectuer un procédé in vitro de détermination d'un paramètre de coagulation, **caractérisée en ce qu'**elle contient un réactif suivant l'une des revendications 1 à 11.

14. Procédé de stabilisation d'un réactif contenant un facteur de tissu et des phospholipides, **caractérisé en ce que** l'on ajoute au réactif au moins un polyphénol soluble dans l'eau, qui a au moins une fonction catéchine, de sorte qu'il est contenu en concentration finale de 0,15 à 10 mM, de manière particulièrement préférée de 0,5 à 2 mM.
